(19) Europäisches Patentamt
European Patent Office
Office européen des brevets

(11) **EP 1 569 155 B1**

(12) **EUROPEAN PATENT SPECIFICATION**

(45) Date of publication and mention
of the grant of the patent:
**11.04.2007 Bulletin 2007/15**

(51) Int Cl.:
***G06F 19/00*** *(2006.01)*

(21) Application number: **05003573.2**

(22) Date of filing: **18.02.2005**

(54) **Method of detecting an error spot in a DNA chip and system using the method**

Verfahren zur Feststellung von falschen Signalen in einem DNA-Chip sowie System zur Verwendung derselben

Méthode de détection d'une erreur de signal dans une puce à ADN et système l'utilisant

(84) Designated Contracting States:
**DE FR GB**

(30) Priority: **21.02.2004 KR 2004011654**

(43) Date of publication of application:
**31.08.2005 Bulletin 2005/35**

(73) Proprietor: **SAMSUNG ELECTRONICS CO., LTD.**
**Suwon-si,**
**Gyeonggi-do (KR)**

(72) Inventor: **Oh, Ji-young**
**Yeongtong-gu**
**Suwon-si**
**Gyeonggi-do (KR)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**WO-A-02/25273          WO-A-20/04068136**

• **KERR K ET AL: "ANALYSIS OF VARIANCE FOR GENE EXPRESSON MICROARRAY DATA" JOURNAL OF COMPUTATIONAL BIOLOGY, MARY ANN LIEBERT, LARCHMONT, NY, US, vol. 7, no. 6, December 2000 (2000-12), pages 819-837, XP009036796 ISSN: 1066-5277**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

1. Field of the Invention

The present invention relates to a method of detecting an error spot and a system using the method, and more specifically, to a method of detecting an error spot by quantifying DNA chips and a system using the method.

2. Description of the Related Art

DNA chips have been manufactured using molecular biological technologies and newly developed mechanical and electronic engineering technologies. DNA chips are chips in which several hundreds to several hundreds of thousands DNAs are integrated in a very small space using mechanical automation and electronic control technologies. That is, DNA chips are chips to which many types of DNAs are attached with high density for detecting genes. DNA chips can replace the conventional genetic engineering technologies, such as southern blotting and northern blotting, mutant detection, and DNA sequencing.

DNA chips are classified into four groups depending on the manufacturing method; pin microarray chips manufactured by micro dotting (surface contact) using a pin, inkjet chips manufactured by micro dotting using an inkjet technology, photolithography chips, and electronic array chips.

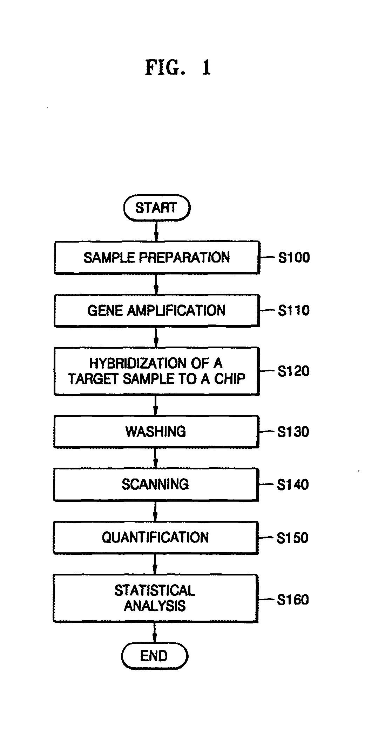

FIG. 1 is a flowchart illustrating a conventional method of analyzing genes using a DNA chip.

Referring to FIG. 1, a sample preparation is performed for taking a sample, i.e., gene to be analyzed (operation (S100)). In the sample preparation, pure genes to be analyzed are extracted from a biological sample, such as blood.

Next, genes extracted via the sample preparation are amplified to an analyzable level (operation (S110)). The amplification operation is generally performed by a polymerase chain reaction (PCR).

Then, the amplified genes, which are a target sample, are hybridized in the DNA chip (operation (S120)). In the hybridization operation, the target sample to be tested is reacted with oligo samples having information of genes and immobilized on the chip. Thus the target sample is hybridized with an oligo sample having a complementary sequence.

Next, a non-hybridized target sample which remains on the chip is washed off (operation (S130)). Then, the image of the chip is scanned by a scanner to detect a degree of hybridization of the target sample with the oligo probe (operation (S140)). Subsequently, the scanned image is quantified for a statistical analysis (operation (S150)).

After quantifying the image of the DNA chip, a statistical analysis is performed using various algorithms and the quantified value of each spot on the chip is analyzed in order to discriminate whether the target sample is originated from a sick person or a normal person (operation (S160)).

As illustrated in FIG. 1, the conventional method of analyzing genes comprises a series of continuous seven operations. During experiments between the first operation and the fifth operation (operations (S100 through S140)), various error factors and thus various types of error spots are generated. If the quantification operation is performed based on false information due to the errors and the statistical analysis is performed using the quantified false data, the false spot data may reduce a reliability of the analysis and limit the possibility to identify a sick person.

SUMMARY OF THE INVENTION

The present invention provides a method of detecting an error spot, which increases a reliability in a statistical analysis by detecting the error spot in a DNA chip and excluding the detected error spot in the statistical analysis and a system using the method.

The present invention also provides a computer-readable recording medium having recorded therein a computer program for executing in a computer a method of detecting an error spot, the method increasing a reliability in a statistical analysis by detecting the error spot in a DNA chip and excluding the detected error spot in the statistical analysis.

According to an aspect of the present invention, there is provided a method of detecting an error spot, comprising the operations of: analyzing a difference in variances for a background intensity and a foreground intensity for each spot in a DNA chip; verifying if a mean of the background intensity and a mean of the foreground intensity are significantly different from each other, based on the difference in variances; and judging an error spot based on the results of the verifying operation.

According to another aspect of the present invention, there is provided a system for detecting an error spot, comprising: a variance analysis part for analyzing a difference in variances for background intensity and a foreground intensity for each spot in a DNA chip; a mean verifying part for verifying whether a mean of the background intensity and a mean of the foreground intensity are significantly different from each other, based on the difference in variances; and an error spot judging part for judging an error spot based on the results of the verifying operation.

According to still another aspect of the present invention, there is provided a computer-readable recording medium having recorded thereto a computer program for executing in a computer a method of detecting an error spot, the method comprising the operations of: analyzing a difference in variances for a background intensity and a foreground intensity for each spot in a DNA chip; verifying whether a mean of the background intensity and a mean of the foreground intensity are significantly different from each other based on the difference in variances; and judging an error spot based on the results of the verifying operation.

BRIEF DESCRIPTION OF THE DRAWINGS

The above and other features and advantages of the present invention will become more apparent by describing in detail exemplary embodiments thereof with reference to the attached drawings in which:

FIG. 1 is a flowchart illustrating a conventional method of analyzing genes using a DNA chip;
FIG. 2 is a flowchart illustrating an image processing procedure for a DNA chip;
FIG. 3 is a diagram illustrating an image scanning of a DNA chip;
FIG. 4 is a diagram illustrating errors generated during analyzing a DNA chip and the types of scanning errors corresponding to the errors generated during analyzing the DNA chip;
FIG. 5 is a diagram illustrating results generated from the types of scanning errors in FIG. 4;
FIG. 6A is a graph illustrating the relationship between a spot size and a spot intensity;
FIG. 6B is a graph illustrating the relationship between a spot intensity and its standard deviation;
FIGS. 7A and 7B are diagrams illustrating input data used in a method of detecting an error spot according to an embodiment of the present invention;
FIG. 8 is a flowchart illustrating a method of detecting an error spot according to an embodiment of the present invention;
FIG. 9 is a block diagram illustrating a system for detecting an error spot according to another embodiment of the present invention;
FIGS. 10 and 11 are diagrams illustrating the ratio and the type of error spots detected in each DNA chip; and
FIG. 12 is a diagram illustrating a change of Robust M caused by excluding error spots.

DETAILED DESCRIPTION OF THE INVENTION

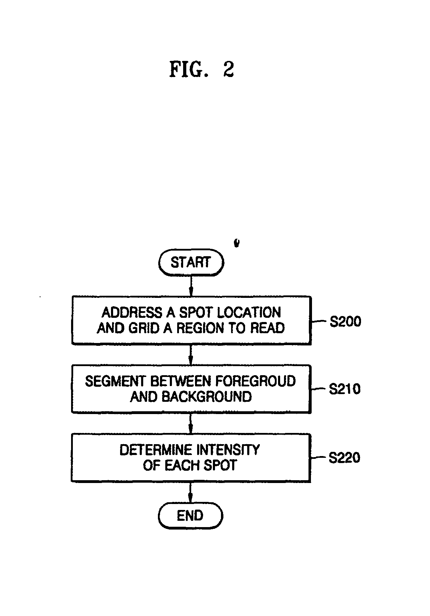

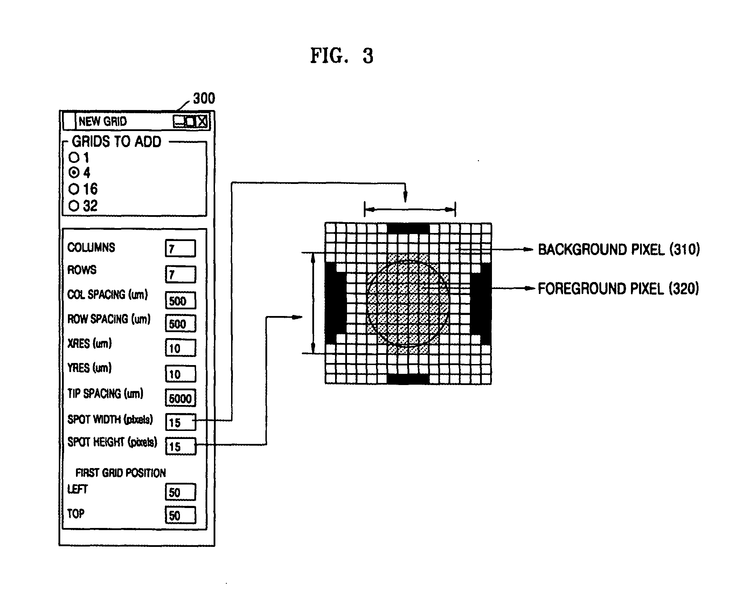

FIG. 2 is a flowchart illustrating an image processing procedure for a DNA chip and FIG. 3 is a diagram illustrating an image scanning of a DNA chip.
In general, the image processing procedure of a DNA chip includes a scanning operation and a quantification operation. The scanning operation and the quantification operation are closely related to each other. Values obtained from the quantification operation change depending on a scanning method.
Referring to FIGS. 2 and 3, there is first performed addressing of a location and a shape of each spot in the DNA chip and gridding of a region to be read (operation (S200)).
Next, a segmentation is performed (operation (210)) in which pixels belonging to a background region (310) and pixels belonging to a foreground region (320) in the addressed spot are segmented. Various methods have been proposed to segment the foreground (320) and the background (310). Representative methods include a fixed circle assumption and an adaptive circle assumption.
The fixed circle assumption method segments a background and a foreground by plotting identical circles for each spot, on the assumption that all spots have the same size and shape. The adaptive circle assumption plots a shape of a spot by connecting pixels having an intensity remarkably different from adjacent pixels, by taking it into account that each spot may have a different shape and a different size.
After segmenting the background and the foreground (operation (S210)), a median value of the intensity is read for each pixel in the background and the foreground, respectively, and the median values are summed up and then divided by the number of pixels to obtain a mean of the intensity for the background and the foreground, respectively. In addition, a standard deviation for the background and the foreground, respectively, is obtained based on the median values of the intensity for each pixel.
Also, various methods of quantifying an intensity by scanning the spot are disclosed. Representative quantifying methods include a method using a standard deviation of a background, a method using a spotted area, and a method using a center point.
The method using a standard deviation of a background is performed based on the percentage of pixels in a foreground, a median intensity of each pixel, which is larger than a median intensity for a background, being added to one or two times its standard deviation. This method is sensitive to the standard deviation of the intensity. However, it is difficult to determine a critical value of the percentage and discriminate an error in alignment and a spot shape.

The method using a spotted area discriminates an error spot by comparing the area of a foreground with the area of gridded region in the spot.

$$\text{Spot shape QC score} = (\text{spot area}=pR^2/2pR)/(\text{spot perimeter}=R/2)$$

If QC score ≤ R/2, the spot is regarded as an error spot.

That is, as a result of the above comparison of the areas, if the area of a foreground is less than R/2, the spot is regarded as an error spot. However, this method cannot distinguish the errors, such as intensity error, spot spreading, non-uniformity of a background and the like.

The method using the center point of a spot comprises comparing the differences between the center point of a spot which was gridded in an immobilized state and the center point of a spot which was gridded in a flexible state and classifying spots having a considerable difference as error spots. However, this method cannot distinguish the errors, such as intensity error, spot spreading and the like.

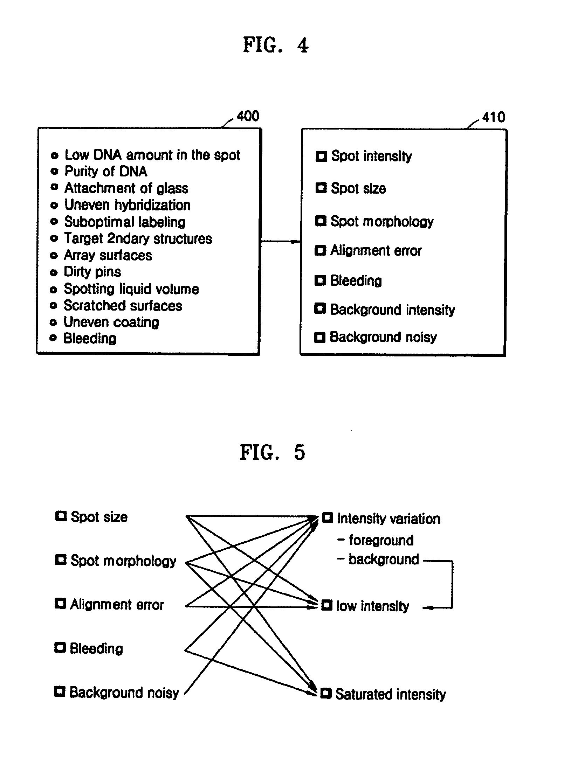

FIG. 4 is a diagram illustrating errors generated during analyzing a DNA chip and the types of scanning errors corresponding to the errors generated during analyzing the DNA chip.

Referring to FIG. 4, the errors (400) generated during analyzing a DNA chip include (1) low DNA amount in the spot, (2) purity of DNA, (3) attachment of glass, (4) uneven hybridization, (5) suboptimal labeling, (6) target 2ndary structures, (7) array surfaces, (8) dirty pins, (9) spotting liquid volume, (10) scratched surfaces, (11) uneven coating, (12) bleeding and the like.

The types of the corresponding scanning errors generated from the errors (400) include (1) spot intensity, (2) spot size, (3) spot morphology, (4) alignment error, (5) bleeding, (6) background intensity, (7) background noisy and the like.

FIG. 5 is a diagram illustrating results resulting from the types of scanning errors as illustrated in FIG. 4.

Referring to FIG. 5, intensity variation results from the errors of spot size, spot morphology, alignment error, bleeding, and background noisy. Low intensity results from the errors of spot size, spot morphology, alignment error, and bleeding. Further, saturated intensity results from the errors of spot size, spot morphology, and bleeding.

Thus, as a result of analyzing the relationship between the error types in the DNA chip and results thereof, the error types are classified as spots exhibiting (1) low intensity, (2) intensity variation in the foreground and the background, or (3) saturated intensity.

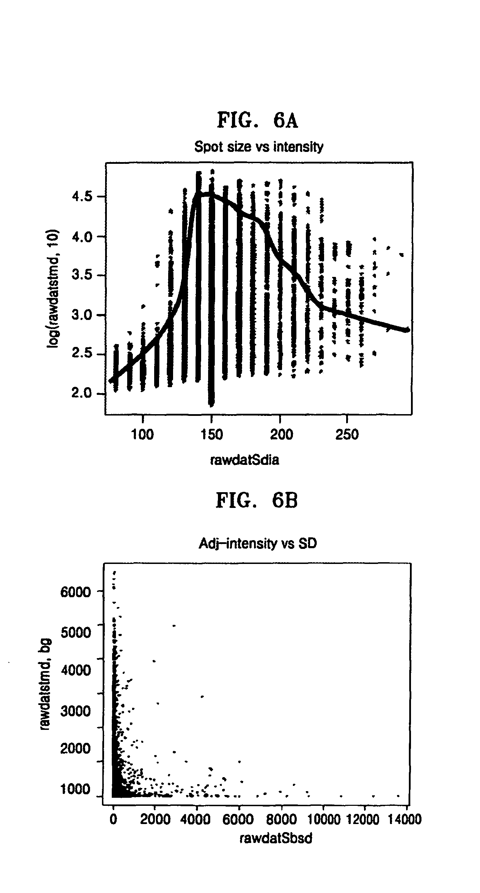

FIG. 6A is a graph illustrating the relationship between a spot size and a spot intensity, and FIG. 6B is a graph illustrating the relationship between a spot intensity and its standard deviation.

Referring to FIG. 6B, the statistical result is that as the deviation of the intensity is higher, a possibility that the intensity is low is higher.

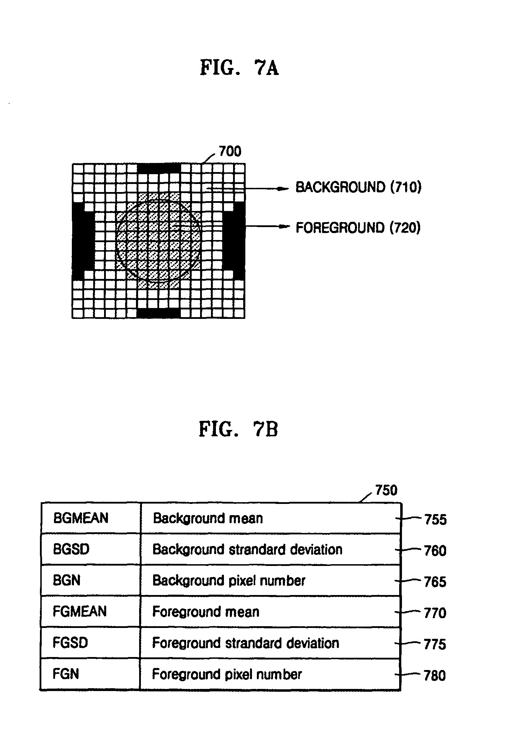

FIGS. 7A and 7B are diagrams illustrating examples of input data used in a method of detecting an error spot according to an embodiment of the present invention.

Referring to FIGS. 7A and 7B, the spots (700) are segmented into the foreground (720) and the background (710). Then, a foreground mean (770) is obtained by dividing a median of intensity of each pixel comprising the foreground (720) by the foreground pixel number (780). And a foreground standard deviation (775) is obtained from the foreground mean (770). Also, a background mean (775) is obtained by dividing a median of intensity of each pixel comprising the background (710) by the background pixel number (765). And a background standard deviation (760) is obtained from the background mean (775).

Thus, input data (750) used in a method of detecting an error spot according to an embodiment of the present invention consist of the mean (770) and the standard deviation (775) for the foreground intensity and the foreground pixel number (780), and the mean (755) and the standard deviation (760) for the background intensity and the background pixel number (765).

There are many programs for quantifying the spot intensity of the DNA chip, each program showing a mean, standard deviation, and pixel number for the background and the foreground, respectively, as a result of quantification. Thus, if the quantification is possible using a conventional program, variables necessary to perform an embodiment of the present invention may be extracted from the files output as a result of the quantification. In general, the quantification program outputs files with a GPR file extension.

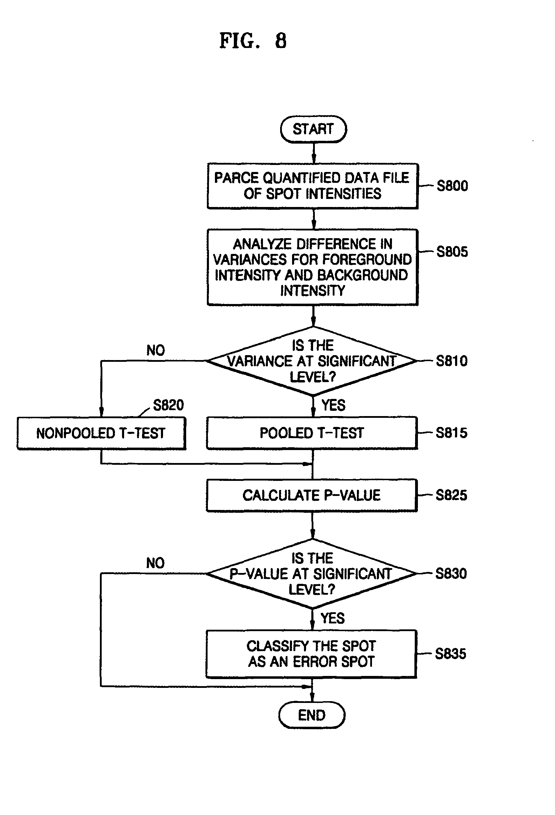

FIG. 8 is a flowchart illustrating a method of detecting an error spot according to an embodiment of the present invention.

Referring to FIG. 8, the quantification program produces an output file including the respective mean, standard deviation, and pixel number for foreground intensity and background intensity of the spot. A conventional quantification program can be used in the embodiment of the present invention.

The output file is subject to parcing (operation (S800)), in order to extract input data consisting of the respective mean, standard deviation, and pixel number for foreground intensity and background intensity of the spot, which are necessary to the present invention from the output file.

Then, the difference in variances is analyzed using the standard deviations for each foreground intensity and background intensity, respectively ((operation (S805)). The f-test is used for analyzing the difference in variances. The f-test is used to verify whether variances of two groups are significantly different from each other.

After the completion of the analysis ((operation (S805)), a verifying operation is performed to establish whether the mean of the background intensity and the mean of the foreground intensity are significantly different from each other, based on the difference in variances ((operations (S810 through S815)). If the results of the difference in variances obtained from the f-test are significant, a pooled t-test is performed for verifying the means. Contrary to this, if the results of the difference in variances obtained from the f-test are not significant, a non-pooled t-test is performed for verifying the means. For example, the resulting value of at least 0.05 in the f-test is judged as being significant, and the resulting value of no more than 0.05 in the f-test is judged as not being significant. The value of 0.05, which is used as a criterion for the establishing the significance, can be somewhat changed depending on the results of statistical results.

The t-test is used to verify whether means of two groups are significantly different or not.

## Equation 1

$$\text{test statistic } t = \frac{(\overline{Y}_1 - \overline{Y}_2) - (\mu_{\gamma 1} - \mu_{\gamma 2})}{S_{\gamma 1 - \gamma 2}} = \frac{\overline{Y}_1 - \overline{Y}_2}{S_{\gamma 1 - \gamma 2}}, \text{ wherein, } H_0 : \mu_{\gamma 1} - \mu_{\gamma 2} = 0 \quad (1)$$

In equation 1, t represents a difference between the means ($\mu_{\gamma 1}$, $\mu_{\gamma 2}$) of the two groups in a pooled t-test, which is used when the two groups have a similar type of deviation.

## Equation 2

$$t = \frac{(\overline{X}_1 - \overline{X}_2) - (\mu_1 - \mu_2)}{\sqrt{\dfrac{(S_1)^2}{n_1} + \dfrac{(S_2)^2}{n_2}}} \qquad (2)$$

## Equation 3

$$\deg ree\ of\ freedom(df) = \frac{[(S_1)^2 / n_1 + (S_2)^2 / n_2]}{[((S_1)^2 /(n_1 - 1)) + ((S_2)^2 /(n_2 - 1))]} \qquad (3)$$

In equation 2, t represents a significant difference between the means of the two groups in a non-pooled t-test, and in equation 3, df represents degrees of freedom. If a variance between the two groups is high, the degrees of freedom are increased, and then the difference between the means is analyzed. Thus, the significant difference between the means is affected by the difference in variances.

After performing the pooled t-test or the non-pooled t-test depending on the difference in variances, a p-value is calculated, based on the result of the pooled or non-pooled t-test (operation (S825). If the p-value is at a significant level, the detected spot is judged as an error spot (operation (S835)). For example, if the p-value is at least 0.05, the p-value is judged as being at significant level and the detected spot is classified as an error spot. The value of 0.05, which is used as a criterion for the judgment of the significance level, can be somewhat changed depending on the results of statistical experimental results.

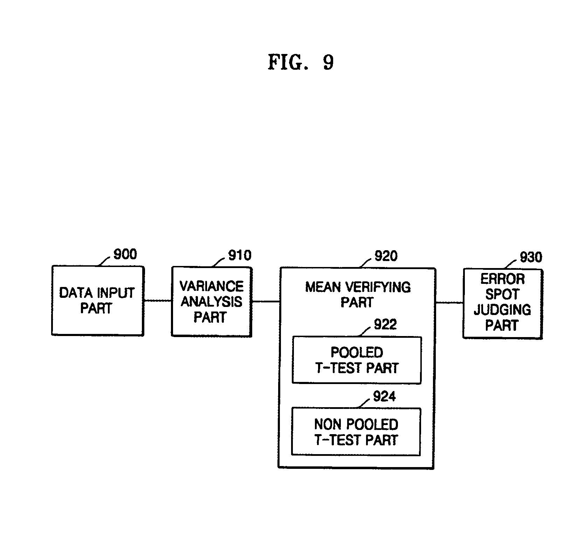

FIG. 9 is a block diagram illustrating a system for detecting an error spot according to an embodiment of the present invention.

Referring to FIG. 9, the system for detecting an error spot is composed of a data input part (900), a variance analysis part (910), a mean verifying part (920) and an error spot judging part (930). The mean verifying part (920) is composed of a pooled t-test part (922) and a non-pooled t-test part (924) operating corresponding to the difference in variances.

The data input part (900) receives a file including the results of the quantification operation. In addition, the data input part (900) extracts input data which are necessary to detect an error spot, from the file. In an embodiment of the present invention, since analyzing variances and verifying means are performed to detect the error spot, the respective mean, standard deviation, and pixel number for background intensity and foreground intensity are extracted to obtain the input data from the file.

In the variance analysis part (910), analysis of the difference in variances for the background intensity and the foreground intensity is performed based on a standard deviation of the input data extracted in the data input part (900). The analysis of the variance is performed using the f-test.

In the mean verifying part (920), verification is performed whether the mean of the background intensity and the mean of the foreground intensity are significantly different from each other, based on the difference in variances in the variance analysis part (910). The verification is performed using the t-test. The variance analysis part (920) may perform the pooled t-test in a pooled t-test part (922) or the non-pooled t-test in a non-pooled t-test part (924), depending the difference in variances.

For example, if the resulting value in the f-test is at least 0.05, the difference in variances are judged as having a significance, and the non-pooled t-test is performed. If the resulting value in the f-test is no more than 0.05, the pooled t-test is performed.

In the error spot judging part (930), the p-value is calculated based on the results in the mean verifying part (920) and a judgment on an error spot is performed based on the p-value. For example, if the p-value is at least 0.05, the detected spot is classified as an error spot.

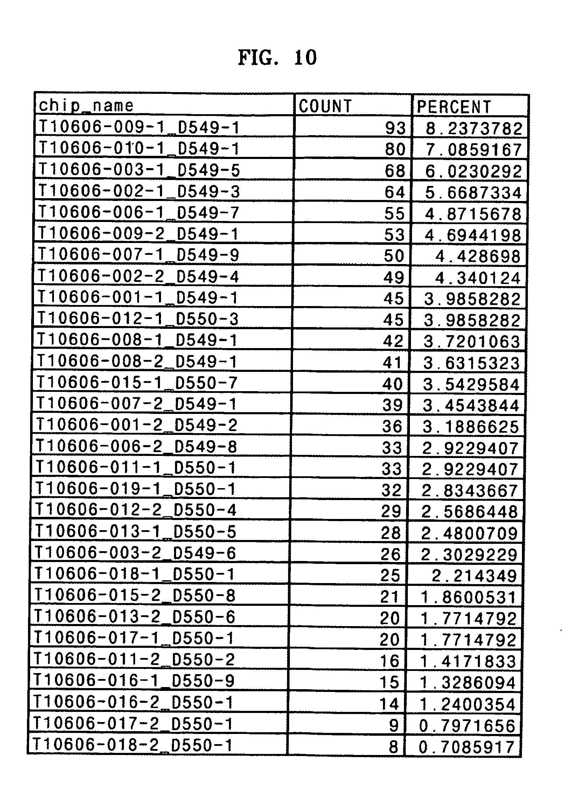

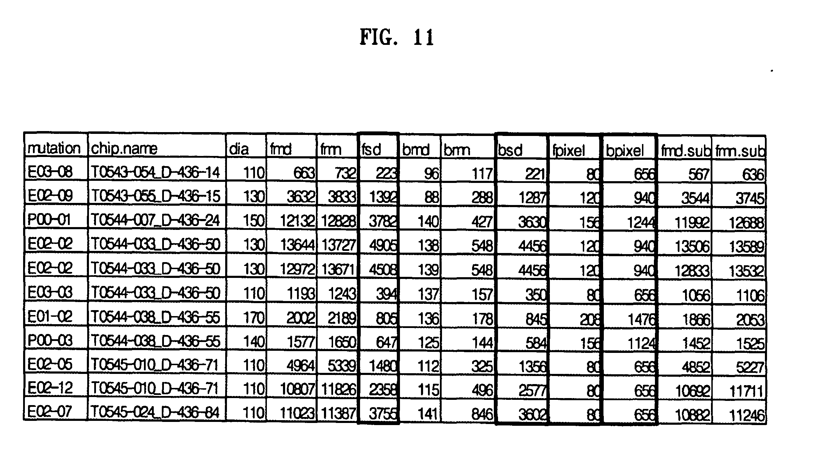

FIGS. 10 and 11 are diagrams illustrating the ratio and the type of error spots detected in each DNA chip.

Referring to FIG. 10, 0.7 to 8.23% of the spots are detected as error spots. As a result of analyzing the data detected as error spots, while in most error spots, the standard deviation of the foreground intensity (fsd) and the standard deviation of the background intensity (bsd) are high and the foreground intensity (fmd) and the background intensity (bmd) are low, some spots having a high standard deviation of the intensity may be detected as error spots even though their intensities are more than 10000.

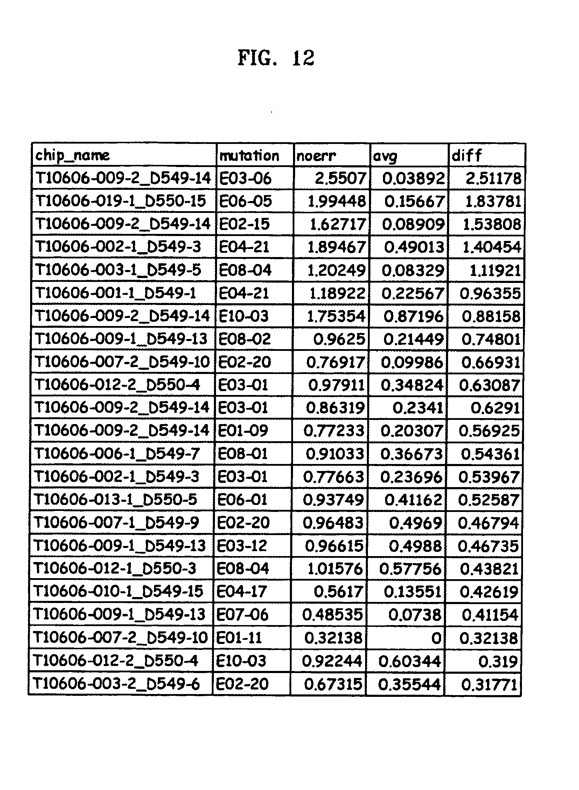

FIG. 12 is a diagram illustrating a change of Robust M caused by excluding error spots.

Referring to FIG. 12, with respect to the change of Robust M, the difference is no more than about 2.5. This is a great difference, taking it into account that if the difference is at least 1 in the analysis, the kernel discriminating the difference changes greatly. Thus, reliability on the results may be increased.

The invention can also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, and carrier waves (such as data transmission through the Internet). The computer readable recording medium can also be distributed over network coupled computer systems so that the computer readable code is stored and executed in a distributed fashion.

According to an embodiment of the present invention, spots having high difference in variances for the foreground intensity and the background intensity are detected as error spots (such as, spots having low intensity resulting from small spot size or incorrect alignment, or spots having partially saturated intensity) and excluded, and thus in the subsequent statistical analysis, errors in discriminating between a sample from a normal person and a sample from a patient can be decreased. That is to say, the reliability in statistical analysis can be increased.

## Claims

1. A method of detecting an error spot, comprising the operations of:

analyzing a difference in variances for a background intensity and a foreground intensity for each spot in a DNA chip;

verifying if a mean of the background intensity and a mean of the foreground intensity are significantly different from each other, based on the difference in variances; and

judging an error spot based on the results of the verifying operation.

2. The method of claim 1, wherein the operation of analyzing the difference in variances comprises performing an f-test based on each standard deviation of the background intensity and the foreground intensity.

3. The method of claim 1, wherein the operation of verifying the means comprises performing a pooled t-test or a non-pooled t-test, based on the difference in variances.

4. The method of claim 1 or 3, wherein the operation of verifying the means comprises increasing degrees of freedom if the difference in variances is high.

5. The method of claim 1, wherein the operation of judging an error spot is based on a p-value calculated from the results of the operation of verifying the significant difference of the means.

6. The method of claim 5, wherein in the operation of judging the error spot, a spot is judged as the error spot if the p-value is at least 0.05.

7. The method of claim 1, further comprising the operation of receiving resultant files generated from a quantifying process and parcing the resultant files to extract input data which are necessary in the operations of analyzing the difference in variances and verifying the means.

8. The method of claim 7, wherein the input data include a first mean and a first standard deviation of the background intensity, the number of pixels in the background, a second mean and a second standard deviation of the foreground intensity, and the number of pixels in the foreground.

9. A system for detecting an error spot, comprising:

a variance analysis part for analyzing a difference in variances for background intensity and a foreground intensity for each spot in a DNA chip;
a mean verifying part for verifying whether a mean of the background intensity and a mean of the foreground intensity are significantly different from each other, based on the difference in variances; and
an error spot judging part for judging an error spot based on the results of the verifying operation.

10. The system of claim 9, further comprising a data input part for receiving resultant files generated from a quantifying process and parcing the resultant files to extract input data which are necessary in the operations of analyzing the difference in variances and verifying the means.

11. The system of claim 10, wherein the input data include a first mean and a first standard deviation of the background intensity, the number of pixels in the background, a second mean and a second standard deviation of the foreground intensity, and the number of pixels in the foreground.

12. The system of claim 9, wherein the variance analysis part analyzes the difference in variances by performing an f-test based on each standard deviation of the background intensity and the foreground intensity.

13. The system of claim 9, wherein the mean verifying part verifies the significant difference of the means by performing a pooled t-test or a non-pooled t-test based on the difference in variances.

14. The system of claim 9, wherein the error spot judging part judges the error spot based on a p-value calculated from the results in the mean verifying part.

15. A computer-readable recording medium having recorded thereto a computer program for executing in a computer a method of detecting an error spot, the method comprising the operations of:

analyzing a difference in variances for a background intensity and a foreground intensity for each spot in a DNA chip;
verifying whether a mean of the background intensity and a mean of the foreground intensity are significantly different from each other based on the difference in variances; and
judging an error spot based on the results of the verifying operation.

**Patentansprüche**

1. Ein Verfahren zum Nachweis eines fehlerhaften Signals umfassend die Operationen von:

   Analysieren eines Unterschieds in den Varianzen für eine Hintergrund-Intensität und eine Vordergrund-Intensität für jedes Signal in einem DNA-Chip;
   Verifizieren, ob ein Mittelwert der Hintergrund-Intensität und ein Mittelwert der Vordergrund-Intensität signifikant voneinander unterschiedlich sind, basierend auf dem Unterschied in den Varianzen; und
   Beurteilen eines fehlerhaften Signals basierend auf den Ergebnissen der verifizierenden Operation.

2. Das Verfahren gemäß Anspruch 1, wobei die Operation des Analysierens des Unterschiedes in den Varianzen das Durchführen eines f-Tests basierend auf jeder Standardabweichung der Hintergrund-Intensität und der Vordergrund-Intensität umfasst.

3. Das Verfahren gemäß Anspruch 1, wobei die Operation des Verifizierens der Mittelwerte das Durchführen eines gepoolten t-Tests oder eines nicht-gepoolten t-Tests umfasst, basierend auf dem Unterschied in den Varianzen.

4. Das Verfahren gemäß Anspruch 1 oder 3, wobei die Operation des Verifizierens der Mittelwerte das Erhöhen der Freiheitsgrade umfasst, falls der Unterschied in den Varianzen hoch ist.

5. Das Verfahren gemäß Anspruch 1, wobei die Operation des Beurteilens eines fehlerhaften Signals auf einem p-Wert basiert, berechnet aus den Ergebnissen der Operation des Verifizierens des signifikanten Unterschiedes der Mittelwerte.

6. Das Verfahren gemäß Anspruch 5, wobei in der Operation des Beurteilens des fehlerhaften Signals ein Signal als das fehlerhafte Signal bewertet wird, falls der p-Wert zumindest 0,05 ist.

7. Das Verfahren gemäß Anspruch 1, desweiteren umfassend die Operation des Empfangens von Resultaten-Dateien, erzeugt aus einem Quantifizierungs-Prozess und das Analysieren der resultierenden Dateien, um Eingabedaten zu extrahieren, welche notwendig sind, in den Operationen des Analysierens des Unterschiedes in den Varianzen sowie beim Verifizieren der Mittelwerte.

8. Das Verfahren gemäß Anspruch 7, wobei die Eingabedaten einen ersten Mittelwert und eine erste Standardabweichung der Hintergrund-Intensität einschließen, die Anzahl der Pixel in dem Hintergrund, einen zweiten Mittelwert und eine zweite Standardabweichung der Vordergrund-Intensität sowie die Anzahl der Pixel in dem Vordergrund.

9. Ein System zum Nachweis eines fehlerhaften Signals umfassend:

   einen Varianz-Analyse-Teil zum Analysieren eines Unterschiedes in den Varianzen für eine Hintergrund-Intensität und eine Vordergrund-Intensität für jedes Signal in einem DNA-Chip;
   einen Mittelwert verifizierenden Teil zum Verifizieren, ob ein Mittelwert der Hintergrund-Intensität und ein Mittelwert der Vordergrund-Intensität signifikant voneinander unterschiedlich sind, basierend auf dem Unterschied in den Varianzen; und
   einen das fehlerhafte Signal beurteilenden Teil zum Auswerten eines fehlerhaften Signals basierend auf den Ergebnissen der verifizierenden Operation.

10. Das System gemäß Anspruch 9, desweiteren umfassend einen Daten-EingabeTeil zum Entgegennehmen von resultierenden Dateien, erzeugt aus einem Quantifizierungs-Prozess und Analysieren der resultierenden Dateien, um Eingabedaten zu extrahieren, welche notwendig sind, in den Operationen des Analysierens des Unterschiedes in den Varianzen und des Verifizierens der Mittelwerte.

11. Das System gemäß Anspruch 10, wobei die Eingabedaten einen ersten Mittelwert und eine erste Standardabweichung der Hintergrund-Intensität einschließen, die Anzahl der Pixel in dem Hintergrund, einen zweiten Mittelwert und eine zweite Standardabweichung der Vordergrund-Intensität und die Anzahl der Pixel in dem Vordergrund.

12. Das System gemäß Anspruch 9, wobei der Varianz-Analyse-Teil den Unterschied in den Varianzen analysiert durch Durchführen eines f-Tests, basierend auf jeder Standardabweichung der Hintergrund-Intensität und der Vordergrund-Intensität.

**13.** Das System gemäß Anspruch 9, wobei der Mittelwert verifizierende Teil den signifikanten Unterschied der Mittelwerte verifiziert durch Durchführen eines gepoolten t-Tests oder eines nicht gepoolten t-Tests, basierend auf den Unterschieden in Varianzen.

**14.** Das System gemäß Anspruch 9, wobei der das fehlerhafte Signal beurteilende Teil das fehlerhafte Signal beurteilt, basierend auf einem p-Wert, berechnet aus den Ergebnissen in dem Mittelwert verifizierenden Teil.

**15.** Ein computerlesbares Aufzeichnungsmedium, welches darauf aufgezeichnet ein Computerprogramm aufweist zum Durchführen eines Verfahrens des Nachweisens eines fehlerhaften Signals in einem Computer, wobei das Verfahren die folgenden Operationen umfasst:

Analysieren eines Unterschieds in den Varianzen für eine Hintergrund-Intensität und eine Vordergrund-Intensität für jedes Signal in einem DNA-Chip;
Verifizieren, ob ein Mittelwert der Hintergrund-Intensität und ein Mittelwert der Vordergrund-Intensität signifikant voneinander unterschiedlich sind, basierend auf dem Unterschied in den Varianzen; und
Beurteilen eines fehlerhaften Signals, basierend auf den Ergebnissen der verifizierenden Operation.

**Revendications**

**1.** Procédé de détection d'erreur de spot, comprenant les opérations consistant à :

analyser une différence de variance entre une intensité d'arrière-plan et une intensité d'avant-plan pour chaque spot d'une puce à ADN ;
vérifier si une moyenne de l'intensité d'arrière-plan et une moyenne de l'intensité d'avant-plan diffèrent de façon importante l'une de l'autre, en se basant sur la différence des variances ; et
estimer une erreur de spot en se basant sur les résultats de l'opération de vérification.

**2.** Procédé selon la revendication 1, dans lequel l'opération d'analyse de la différence des variances consiste à effectuer un test f en se basant sur chaque écart-type de l'intensité d'arrière-plan et de l'intensité d'avant-plan.

**3.** Procédé selon la revendication 1, dans lequel l'opération de vérification des moyennes consiste à effectuer un test t combiné ou un test t non combiné, en se basant sur la différence des variances.

**4.** Procédé selon la revendication 1 ou 3, dans lequel l'opération de vérification des moyennes consiste à augmenter des degrés de liberté si la différence des variances est élevée.

**5.** Procédé selon la revendication 1, dans lequel l'opération d'estimation d'erreur de spot est basée sur une valeur p calculée à partir des résultats de l'opération de vérification de l'importance de la différence des moyennes.

**6.** Procédé selon la revendication 5, dans lequel dans l'opération d'estimation d'erreur de spot, un spot est estimé comme étant le spot erroné si la valeur p est égale à au moins 0,05.

**7.** Procédé selon la revendication 1, comprenant en outre l'opération qui consiste à recevoir des fichiers résultants générés à partir d'un procédé de quantification et à analyser ces fichiers résultants pour extraire des données d'entrée qui sont nécessaires aux opérations d'analyse de la différence des variances et de vérification des moyennes.

**8.** Procédé selon la revendication 7, dans lequel les données d'entrée comprennent une première moyenne et un premier écart-type de l'intensité d'arrière-plan, le nombre de pixels de l'arrière-plan, une seconde moyenne et un second écart-type de l'intensité d'avant-plan, et le nombre de pixels de l'avant-plan.

**9.** Système de détection d'erreur de spot, comprenant :

une partie d'analyse des variances pour analyser une différence de variance entre une intensité d'arrière-plan et une intensité d'avant-plan pour chaque spot d'une puce à ADN ;
une partie de vérification des moyennes pour vérifier si une moyenne de l'intensité d'arrière-plan et une moyenne de l'intensité d'avant-plan diffèrent de façon importante l'une de l'autre, en se basant sur la différence des variances ; et

une partie d'estimation d'erreur de spot pour estimer une erreur de spot en se basant sur les résultats de l'opération de vérification.

**10.** Système selon la revendication 9, comprenant en outre une partie d'entrée de données pour recevoir des fichiers résultants générés à partir d'un procédé de quantification et analyser ces fichiers résultants pour extraire des données d'entrée qui sont nécessaires aux opérations d'analyse de la différence des variances et de vérification des moyennes.

**11.** Système selon la revendication 10, dans lequel les données d'entrée comprennent une première moyenne et un premier écart-type de l'intensité d'arrière-plan, le nombre de pixels de l'arrière-plan, une seconde moyenne et un second écart-type de l'intensité d'avant-plan, et le nombre de pixels de l'avant-plan.

**12.** Système selon la revendication 9, dans lequel la partie d'analyse des variances analyse la différence des variances en effectuant un test f en se basant sur chaque écart-type de l'intensité d'arrière-plan et de l'intensité d'avant-plan.

**13.** Système selon la revendication 9, dans lequel la partie de vérification des moyennes vérifie l'importance de la différence des moyennes en effectuant un test t combiné ou un test t non combiné en se basant sur la différence des variances.

**14.** Système selon la revendication 9, dans lequel la partie d'estimation d'erreur de spot estime l'erreur de spot en se basant sur une valeur p calculée à partir des résultats de la partie de vérification des moyennes.

**15.** Support d'enregistrement lisible par un ordinateur, ayant enregistré sur celui-ci un programme informatique pour exécuter dans un ordinateur un procédé de détection d'erreur de spot, le procédé comprenant les opérations consistant à :

analyser une différence de variance entre une intensité d'arrière-plan et une intensité d'avant-plan pour chaque spot d'une puce à ADN ;

vérifier si une moyenne de l'intensité d'arrière-plan et une moyenne de l'intensité d'avant-plan diffèrent de façon importante l'une de l'autre, en se basant sur la différence des variances ; et

estimer une erreur de spot en se basant sur les résultats de l'opération de vérification.

# FIG. 1

```
            ( START )
                │
                ▼
┌─────────────────────────────┐
│      SAMPLE PREPARATION      │──── S100
└─────────────────────────────┘
                │
                ▼
┌─────────────────────────────┐
│      GENE AMPLIFICATION      │──── S110
└─────────────────────────────┘
                │
                ▼
┌─────────────────────────────┐
│       HYBRIDIZATION OF A     │──── S120
│    TARGET SAMPLE TO A CHIP   │
└─────────────────────────────┘
                │
                ▼
┌─────────────────────────────┐
│           WASHING            │──── S130
└─────────────────────────────┘
                │
                ▼
┌─────────────────────────────┐
│           SCANNING           │──── S140
└─────────────────────────────┘
                │
                ▼
┌─────────────────────────────┐
│        QUANTIFICATION        │──── S150
└─────────────────────────────┘
                │
                ▼
┌─────────────────────────────┐
│          STATISTICAL         │──── S160
│           ANALYSIS           │
└─────────────────────────────┘
                │
                ▼
            (  END  )
```

# FIG. 2

```
        ( START )

┌─────────────────────────────┐
│  ADDRESS A SPOT LOCATION     │──── S200
│  AND GRID A REGION TO READ   │
└─────────────────────────────┘

┌─────────────────────────────┐
│  SEGMENT BETWEEN FOREGROUD   │──── S210
│  AND BACKGROUND              │
└─────────────────────────────┘

┌─────────────────────────────┐
│  DETERMINE INTENSITY         │──── S220
│  OF EACH SPOT                │
└─────────────────────────────┘

         ( END )
```

# FIG. 3

300

| NEW GRID | ▭◻✕ |

**GRIDS TO ADD**
- ○ 1
- ⊙ 4
- ○ 16
- ○ 32

| COLUMNS | 7 |
| ROWS | 7 |
| COL SPACING (um) | 500 |
| ROW SPACING (um) | 500 |
| XRES (um) | 10 |
| YRES (um) | 10 |
| TIP SPACING (um) | 5000 |
| SPOT WIDTH (pixels) | 15 |
| SPOT HEIGHT (pixels) | 15 |

FIRST GRID POSITION
| LEFT | 50 |
| TOP | 50 |

→ BACKGROUND PIXEL (310)

→ FOREGROUND PIXEL (320)

EP 1 569 155 B1

# FIG. 4

**400**

- Low DNA amount in the spot
- Purity of DNA
- Attachment of glass
- Uneven hybridization
- Suboptimal labeling
- Target 2ndary structures
- Array surfaces
- Dirty pins
- Spotting liquid volume
- Scratched surfaces
- Uneven coating
- Bleeding

**410**

- Spot intensity
- Spot size
- Spot morphology
- Alignment error
- Bleeding
- Background intensity
- Background noisy

# FIG. 5

- Spot size
- Spot morphology
- Alignment error
- Bleeding
- Background noisy

- Intensity variation
  - foreground
  - background
- low intensity
- Saturated intensity

## FIG. 6A

Spot size vs intensity

## FIG. 6B

Adj-intensity vs SD

## FIG. 7A

700

BACKGROUND (710)

FOREGROUND (720)

## FIG. 7B

750

| BGMEAN | Background mean | 755 |
|--------|-----------------|-----|
| BGSD | Background strandard deviation | 760 |
| BGN | Background pixel number | 765 |
| FGMEAN | Foreground mean | 770 |
| FGSD | Foreground strandard deviation | 775 |
| FGN | Foreground pixel number | 780 |

## FIG. 8

```
                    ( START )
                        │
                        ▼
            ┌───────────────────────┐
            │ PARCE QUANTIFIED DATA FILE │──── S800
            │    OF SPOT INTENSITIES     │
            └───────────────────────┘
                        │
                        ▼
            ┌───────────────────────┐
            │    ANALYZE DIFFERENCE IN   │──── S805
            │  VARIANCES FOR FOREGROUND  │
            │   INTENSITY AND BACKGROUND │
            │         INTENSITY          │
            └───────────────────────┘
                        │
                        ▼
                   ╱ IS THE ╲
         NO     ╱ VARIANCE AT SIGNIFICANT ╲──── S810
    ┌──────────╲        LEVEL?        ╱
    │           ╲              ╱
    │                    │ YES
    ▼                    ▼
┌─────────────┐    ┌─────────────┐
│ NONPOOLED   │    │  POOLED T-TEST │──── S815
│  T-TEST     │    └─────────────┘
└─────────────┘          │
  S820   │               │
         └───────────────┤
                         ▼
              ┌─────────────────┐
              │ CALCULATE P-VALUE │──── S825
              └─────────────────┘
                         │
                         ▼
                   ╱ IS THE ╲
         NO     ╱ P-VALUE AT SIGNIFICANT ╲──── S830
    ┌──────────╲        LEVEL?        ╱
    │           ╲              ╱
    │                    │ YES
    │                    ▼
    │         ┌─────────────────┐
    │         │  CLASSIFY THE SPOT │──── S835
    │         │  AS AN ERROR SPOT  │
    │         └─────────────────┘
    │                    │
    └────────────────────┤
                         ▼
                     ( END )
```

FIG. 9

DATA INPUT PART 900 — VARIANCE ANALYSIS PART 910 — MEAN VERIFYING PART 920 [POOLED T-TEST PART 922, NON POOLED T-TEST PART 924] — ERROR SPOT JUDGING PART 930

## FIG. 10

| chip_name | COUNT | PERCENT |
|---|---|---|
| T10606-009-1_D549-1 | 93 | 8.2373782 |
| T10606-010-1_D549-1 | 80 | 7.0859167 |
| T10606-003-1_D549-5 | 68 | 6.0230292 |
| T10606-002-1_D549-3 | 64 | 5.6687334 |
| T10606-006-1_D549-7 | 55 | 4.8715678 |
| T10606-009-2_D549-1 | 53 | 4.6944198 |
| T10606-007-1_D549-9 | 50 | 4.428698 |
| T10606-002-2_D549-4 | 49 | 4.340124 |
| T10606-001-1_D549-1 | 45 | 3.9858282 |
| T10606-012-1_D550-3 | 45 | 3.9858282 |
| T10606-008-1_D549-1 | 42 | 3.7201063 |
| T10606-008-2_D549-1 | 41 | 3.6315323 |
| T10606-015-1_D550-7 | 40 | 3.5429584 |
| T10606-007-2_D549-1 | 39 | 3.4543844 |
| T10606-001-2_D549-2 | 36 | 3.1886625 |
| T10606-006-2_D549-8 | 33 | 2.9229407 |
| T10606-011-1_D550-1 | 33 | 2.9229407 |
| T10606-019-1_D550-1 | 32 | 2.8343667 |
| T10606-012-2_D550-4 | 29 | 2.5686448 |
| T10606-013-1_D550-5 | 28 | 2.4800709 |
| T10606-003-2_D549-6 | 26 | 2.3029229 |
| T10606-018-1_D550-1 | 25 | 2.214349 |
| T10606-015-2_D550-8 | 21 | 1.8600531 |
| T10606-013-2_D550-6 | 20 | 1.7714792 |
| T10606-017-1_D550-1 | 20 | 1.7714792 |
| T10606-011-2_D550-2 | 16 | 1.4171833 |
| T10606-016-1_D550-9 | 15 | 1.3286094 |
| T10606-016-2_D550-1 | 14 | 1.2400354 |
| T10606-017-2_D550-1 | 9 | 0.7971656 |
| T10606-018-2_D550-1 | 8 | 0.7085917 |

FIG. 11

| mutation | chip.name | dia | fmd | frm | fsd | bmd | brm | bsd | fpixel | bpixel | fmd.sub | frm.sub |
|---|---|---|---|---|---|---|---|---|---|---|---|---|
| E03-08 | T0543-054_D-436-14 | 110 | 663 | 732 | 223 | 96 | 117 | 221 | 80 | 656 | 567 | 636 |
| E02-09 | T0543-055_D-436-15 | 130 | 3632 | 3833 | 1392 | 88 | 288 | 1287 | 120 | 940 | 3544 | 3745 |
| P00-01 | T0544-007_D-436-24 | 150 | 12132 | 12828 | 3782 | 140 | 427 | 3630 | 156 | 1244 | 11992 | 12688 |
| E02-02 | T0544-033_D-436-50 | 130 | 13644 | 13727 | 4905 | 138 | 548 | 4456 | 120 | 940 | 13506 | 13589 |
| E02-02 | T0544-033_D-436-50 | 130 | 12972 | 13671 | 4508 | 139 | 548 | 4456 | 120 | 940 | 12833 | 13532 |
| E03-03 | T0544-033_D-436-50 | 110 | 1193 | 1243 | 394 | 137 | 157 | 350 | 80 | 656 | 1056 | 1106 |
| E01-02 | T0544-038_D-436-55 | 170 | 2002 | 2189 | 805 | 136 | 178 | 845 | 208 | 1476 | 1866 | 2053 |
| P00-03 | T0544-038_D-436-55 | 140 | 1577 | 1650 | 647 | 125 | 144 | 584 | 156 | 1124 | 1452 | 1525 |
| E02-05 | T0545-010_D-436-71 | 110 | 4964 | 5339 | 1480 | 112 | 325 | 1356 | 80 | 656 | 4852 | 5227 |
| E02-12 | T0545-010_D-436-71 | 110 | 10807 | 11826 | 2356 | 115 | 496 | 2577 | 80 | 656 | 10692 | 11711 |
| E02-07 | T0545-024_D-436-84 | 110 | 11023 | 11387 | 3755 | 141 | 846 | 3602 | 80 | 656 | 10882 | 11246 |

FIG. 12

| chip_name | mutation | noerr | avg | diff |
|---|---|---|---|---|
| T10606-009-2_D549-14 | E03-06 | 2.5507 | 0.03892 | 2.51178 |
| T10606-019-1_D550-15 | E06-05 | 1.99448 | 0.15667 | 1.83781 |
| T10606-009-2_D549-14 | E02-15 | 1.62717 | 0.08909 | 1.53808 |
| T10606-002-1_D549-3 | E04-21 | 1.89467 | 0.49013 | 1.40454 |
| T10606-003-1_D549-5 | E08-04 | 1.20249 | 0.08329 | 1.11921 |
| T10606-001-1_D549-1 | E04-21 | 1.18922 | 0.22567 | 0.96355 |
| T10606-009-2_D549-14 | E10-03 | 1.75354 | 0.87196 | 0.88158 |
| T10606-009-1_D549-13 | E08-02 | 0.9625 | 0.21449 | 0.74801 |
| T10606-007-2_D549-10 | E02-20 | 0.76917 | 0.09986 | 0.66931 |
| T10606-012-2_D550-4 | E03-01 | 0.97911 | 0.34824 | 0.63087 |
| T10606-009-2_D549-14 | E03-01 | 0.86319 | 0.2341 | 0.6291 |
| T10606-009-2_D549-14 | E01-09 | 0.77233 | 0.20307 | 0.56925 |
| T10606-006-1_D549-7 | E08-01 | 0.91033 | 0.36673 | 0.54361 |
| T10606-002-1_D549-3 | E03-01 | 0.77663 | 0.23696 | 0.53967 |
| T10606-013-1_D550-5 | E06-01 | 0.93749 | 0.41162 | 0.52587 |
| T10606-007-1_D549-9 | E02-20 | 0.96483 | 0.4969 | 0.46794 |
| T10606-009-1_D549-13 | E03-12 | 0.96615 | 0.4988 | 0.46735 |
| T10606-012-1_D550-3 | E08-04 | 1.01576 | 0.57756 | 0.43821 |
| T10606-010-1_D549-15 | E04-17 | 0.5617 | 0.13551 | 0.42619 |
| T10606-009-1_D549-13 | E07-06 | 0.48535 | 0.0738 | 0.41154 |
| T10606-007-2_D549-10 | E01-11 | 0.32138 | 0 | 0.32138 |
| T10606-012-2_D550-4 | E10-03 | 0.92244 | 0.60344 | 0.319 |
| T10606-003-2_D549-6 | E02-20 | 0.67315 | 0.35544 | 0.31771 |